# EUROPEAN PATENT SPECIFICATION

(11) **EP 3 222 610 B1**
(45) Date of publication and mention of the grant of the patent: **05.12.2018**
(21) Application number: 17160764.1
(22) Date of filing: 14.03.2017
(51) Int. Cl.: C07C 209/60, C07C 211/21, C07C 211/27

(54) **METHOD FOR SELECTIVE PALLADIUM-CATALYZED TELOMERIZATION OF SUBSTITUTED DIENES**
VERFAHREN ZUR SELEKTIVEN PALLADIUMKATALYSIERTEN TELOMERISIERUNG VON SUBSTITUIERTEN DIENEN
PROCÉDÉ DE TÉLOMÉRISATION SÉLECTIVE DE DIÈNES SUBSTITUÉS CATALYSÉE PAR LE PALLADIUM

(30) Priority: 23.03.2016 US 201662312074 P
(43) Date of publication of application: 27.09.2017
(73) Proprietor: International Flavors & Fragrances Inc., New York, NY 10019 (US)
(72) Inventor: MIRANDA, Sergio Castillon, 43893 Altafulla (ES); CABRERO, Carmen Claver, 43893 Altafulla (ES); GODARD, Cyril, 43005 Tarragona (ES); BOVE, Jordi Colavida, 43392 Castellvell del Camp (ES); PEREZ, Ana Maria Collado, 12580 Benicarlo (ES); BODEN, Richard M., Ocean, NJ New Jersey 07712 (US); AMORELLI, Benjamin, Brielle, NJ New Jersey (US)
(74) Representative: Barker Brettell LLP

(56) References cited:
- EP-A1- 0 542 366
- EP-A1- 1 201 634
- US-A1- 2005 038 273
- US-A1- 2005 065 387
- US-A1- 2005 240 039
- MATHIEU J-L TSCHAN ET AL: "Efficient bulky phosphines for the selective telomeriation of 1,3-butadiene with methanol", JOURNAL OF THE AMERICAN CHEMICAL SOCIETY, THE SOCIETY, vol. 132, no. 18, 12 May 2010 (2010-05-12) , pages 6463-6473, XP002659016, ISSN: 0002-7863, DOI: 10.1021/JA100521M [retrieved on 2010-04-20]
- NUNES ET AL: "The unique behavior of the catalytic system Pd(OAc)"2/N-heterocyclic carbene on the telomerization of isoprene with methanol", CATALYSIS COMMUNICATIONS, ELSEVIER, AMSTERDAM, NL, vol. 8, no. 11, 1 November 2007 (2007-11-01), pages 1798-1802, XP022262229, ISSN: 1566-7367, DOI: 10.1016/J.CATCOM.2007.01.033
- GROTEVENDT ET AL: "Efficient catalysts for telomerization of butadiene with amines", TETRAHEDRON LETTERS, ELSEVIER, AMSTERDAM, NL, vol. 48, no. 52, 22 October 2007 (2007-10-22), pages 9203-9207, XP022368229, ISSN: 0040-4039, DOI: 10.1016/J.TETLET.2007.10.076
- JACKSTELL ET AL: "Telomerization and dimerization of isoprene by in situ generated palladium-carbene catalysts", JOURNAL OF ORGANOMETALLIC CHEMISTRY, ELSEVIER-SEQUOIA S.A. LAUSANNE, CH, vol. 692, no. 21, 14 September 2007 (2007-09-14), pages 4737-4744, XP022248212, ISSN: 0022-328X, DOI: 10.1016/J.JORGANCHEM.2007.06.039
- HIDAI M ET AL: "Palladium-catalyzed asymmetric telomerization of isoprene. Preparation of optically active citronellol", JOURNAL OF ORGANOMETALLIC CHEMISTRY, ELSEVIER-SEQUOIA S.A. LAUSANNE, CH, vol. 232, no. 1, 1 January 1982 (1982-01-01), pages 89-98, XP027396561, ISSN: 0022-328X [retrieved on 1982-01-01]

## Description

### Background

The telomerization of dienes is a reaction with high atomic efficiency that includes the dimerization of these substrates followed by the addition of a nucleophile. Studies concerning the telomerization of dienes are conventionally carried out using butadiene as the substrate (Consiglio & Waymouth (1989) Chem. Rev. 89:257; Clement, et al. (2008) Chem. Eur. J. 14:7408; van Leeuwen, et al. (2011) Coord. Chem. Rev. 255:1499; Grotevendt, et al. (2007) Tetrahedron Lett. 48:9203; Tschan, et al. (2010) J. Am. Chem. Soc. 132:6463; EP 0542366). Nucleophiles such as alcohols, water or amines are commonly used, and provide a simple and direct method to products such as 1-octanol. The telomerization of unsymmetrical substrates such isoprene has been much less studied as it presents significant regioselectivity challenges (Lapkin, et al. (2015) Catal. Sci. Technol. 5:1206; Jackstell, et al. (2007) J. Organomet. Chem. 692:4737; Nunes, et al. (2007) Catal. Commun. 8:1798; Hidai, et al. (1982) J. Organomet. Chem. 232:89; Dani, et al. (1996) J. Braz. Chem. Soc. 7:15; Keim, et al. (1983) J. Catal. 20:129; Leca & Reau (2006) J. Catal. 238:425; Röper, et al. (1985) J. Mol. Catal. 31:335; Maddock & Finn (2000) Organometallics 19:2684). Indeed, with this substrate, up to twelve products can be obtained, although the linear telomers **1-4 (1'-4')** are usually the main products (Scheme 1). Moreover, four branched telomers **5-8** (**5'-8'**) can also be obtained, in addition to four distinct trienes by-products.

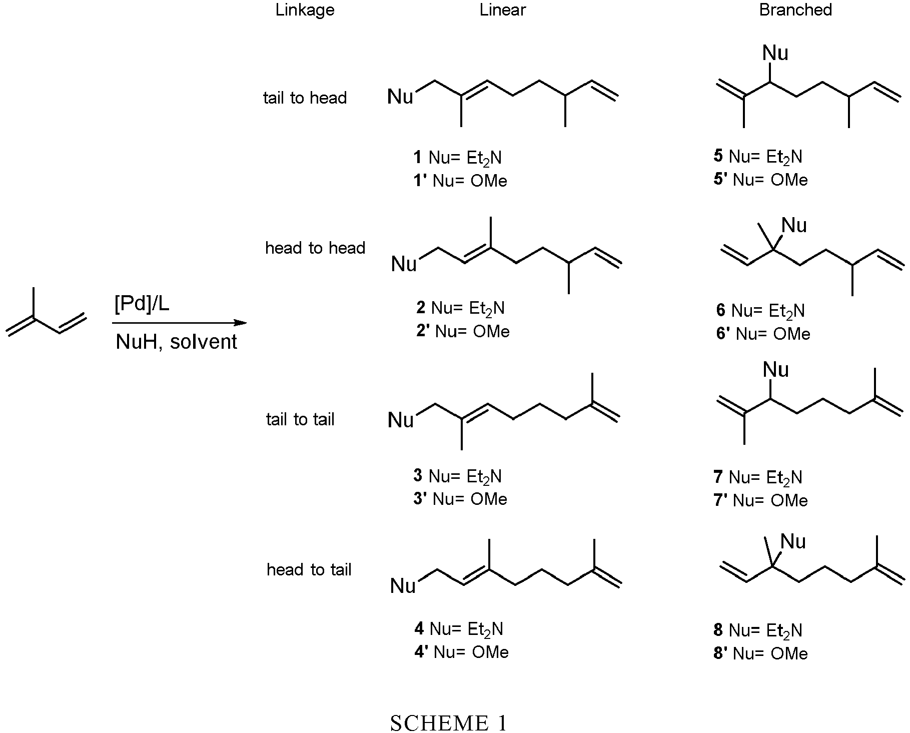

Each active catalytic system requires certain catalyst components: a catalyst precursor and additional ligand and/or other additives, a nucleophile, and solvent, within a specific molar range relative to each other, under a specified set of reaction conditions. The active catalyst is formed in an environment whereby a catalyst precursor is combined with an additional ligand and/or additives to modify the properties of the active catalyst precursor, to create the active catalyst *in-situ.* This new organopalladium catalyst becomes active in the reaction under certain conditions providing novel regioselectivity and yield of telomers from an unsymmetrical diene.

In this invention, particular active catalytic systems provide novel regioselectivity and yield of each of a tail-to-head telomer, a head-to-head telomer, and a tail-to-tail telomer from an unsymmetrical diene substrate.

The catalyst precursor, the ligand and the solvent can play an important role in the yield and regioselectivity of a reaction. Active catalyst precursors used in the telomerization of isoprene can include Pd complexes such as PdCl₂, Pd(OAc)₂, and [Pd(C₃H₅)(COD)]BF₄. Various phosphines, phosphites and phospholes have been used as ligands using diethylamine as the nucleophile. It has been shown that the Pd precursor has an effect on conversion (Maddock & Finn (2000) *supra*). In this work, the cationic π-allyl complex [Pd(C₃H₅)(COD)]BF₄ provided faster telomerization reactions than PdCl₂. Interestingly, trialkylphosphines such as, PEt₃, PnBu₃, PtBu₃ or Pcy₃ showed no activity with PdCl₂, while the use of the cationic complex provided telomerization products in good yields using the same ligands.

The control of the regioselectivity in isoprene telomerization is challenging and there are few catalytic systems providing good regioselectivities for the linear telomers **1-3.** Palladium catalysts bearing various ligands have been reported to provide telomers **1** (tail-to-head; Leca & Réau (2006) *supra*), **2** (head-to-head; Maddock & Finn (2000) *supra*), and **3** (tail-to-tail; Leca & Réau (2006) *supra;* Keim, et al. (1983) *supra*) in moderate-to-good yields and good selectivities using diethylamine as the nucleophile (Scheme 2). Monophosphines, monophosphites and *P,N* ligands were the ligands that provided the highest yields and selectivities.

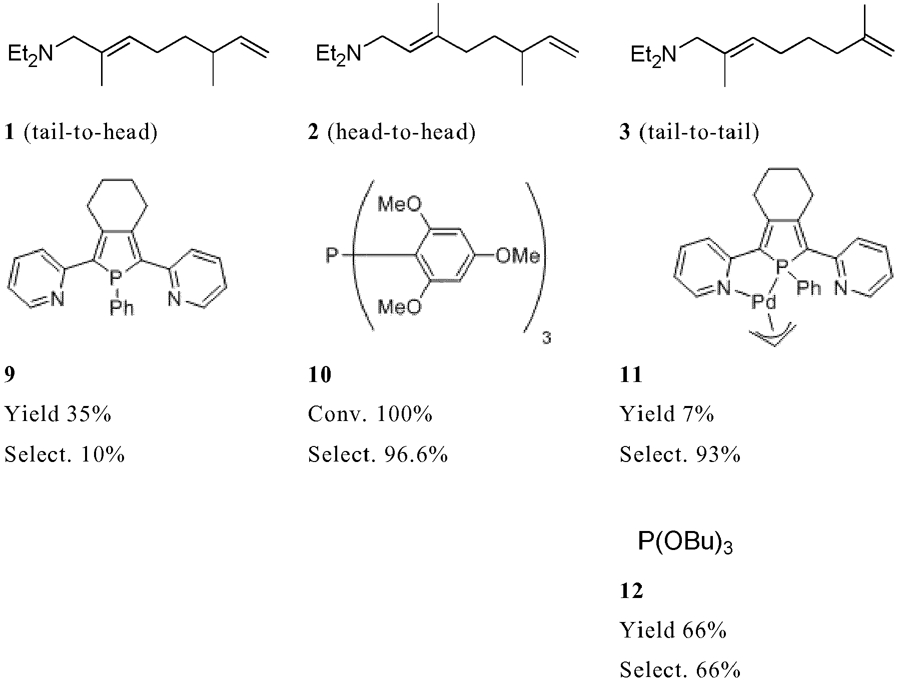

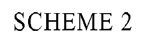

Pd/carbene catalytic systems have also been reported as catalysts in the telomerization of isoprene using sodium methoxide as nucleophile and afforded good yield and moderate selectivities (Navarro, et al. (2015) 5:1447; Jackstell, et al. (2007) *supra;* Nunes, et al. (2007) *supra;* Hidai, et al. (1982) *supra;* Dani, et al. (1996) *supra*). The catalytic system Pd(acac)₂/**13-14** in the presence of base provided the telomer **2'** in 75% selectivity. However, when the bulkier ligands **15-16** were used, the yield decreased significantly. Selectivity of 83% toward telomer **1'** (23% yield) was obtained with the catalytic system Pd(acac)₂/PCy₃.

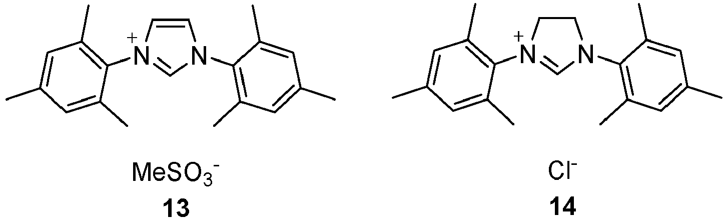

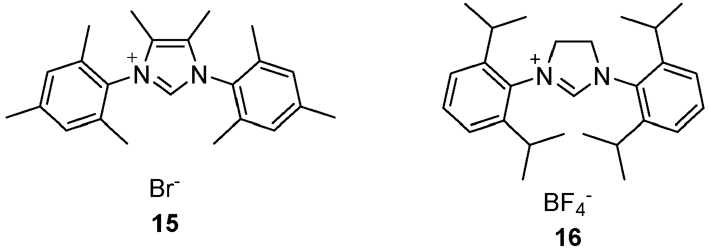

US 2005/0038273 relates to a process for the telomerization of acyclic olefins having at least two conjugated double bonds or mixtures comprising such olefins by means of nulceophiles using a palladium-carbene complex as a catalyst. US 2005/0240039 uses a metal-carbene complex as a catalyst. EP 1201634 relates to a process for telomerizing a conjugated diene which comprises reacting said conjugated diene with a compound containing active hydrogen in the presence of a catalyst system. US 2005/0065387 relates to a process for preparing 1-octene by telomerization of 1,3-butadiene using a reducing agent in the presence of a telomerization catalyst and partial hydrogenation of the octadiene obtained in this way.

While carbenes and a large number of phosphine and phosphite derivatives have been used in telomerization of isoprene, there are very few catalytic systems providing good yields and selectivities. This selectivity issue remains an important challenge in this process and requires efficient solutions. Conventional catalytic systems (*e.g.,* PdCl₂, PPh₃, isoprene, diethylamine, MeOH, room temperature) without special treatment with a unique active catalyst provide poor regioselectivity of telomerization products (51/12/25/8 tail-to-head : head-to-head : tail-to-tail : head-to-tail, respectively).

### Summary of the Invention

The present invention is a method for regioselective synthesis of a telomer from isoprene by polymerizing isoprene in the presence of an active catalytic system containing: (a) an active catalyst precursor and a ligand, wherein (i) the active catalyst precursor is [Pd(C₃H₅)COD]BF₄ or Pd(OAc)₂; and (ii) the ligand is dicyclohexyl-[1-(2,4,6-trimethylphenyl) imidazol-2-yl]phosphane, triphenylphosphine or tris(2,4-di-tert-butylphenyl)phosphite; and (b) a solvent such as methanol, ethanol, propanol, iso-propanol, toluene, acetone, acetonitrile, trifluoroethanol, diethyl ether or tetrahydrofuran. In some embodiments, the active catalyst precursor and the ligand have a molar ratio between 1:3 and 1:0.1. In one embodiment, the active catalyst precursor is [Pd(C₃H₅)COD]BF₄; the ligand is dicyclohexyl-[1-(2,4,6-trimethylphenyl)imidazol-2-yl]phosphane; the solvent is methanol, ethanol, propanol, iso-propanol, toluene, acetone, or acetonitrile and the telomer is a tail-to-head telomere, *e.g.,* with a yield of greater than 60% by weight. In another embodiment, the active catalyst precursor is [Pd(C₃H₅)COD]BF₄; the ligand is triphenylphosphine; the solvent is trifluoroethanol, and the telomer is a head-to-head telomer, *e.g.,* with a yield of greater than 60% by weight. In a further embodiment, the active catalyst precursor is Pd(OAc)₂; the ligand is triphenylphosphine; the solvent is trifluoroethanol, and the telomer is a head-to-head telomer, *e.g.,* with a yield of greater than 60% by weight. In yet a further embodiment, the active catalyst precursor is [Pd(C₃H₅)COD]BF₄; the ligand is tris(2,4-di-tert-butylphenyl)phosphite; the solvent is diethyl ether, tetrahydrofuran or toluene, and the telomer is a tail-to-tail telomer, *e.g.,* with a yield of greater than 60% by weight. In other embodiments, the active catalytic system further contains a nucleophile, *e.g.,* an amine.

### Detailed Description of the Invention

By screening Pd precursors, phosphines, solvents and reaction conditions, a new catalytic system, *i.e.,* [Pd(C₃H₅)COD]BF₄/**22** in the presence of MeOH, was found to provide excellent yields and selectivities of the tail-to-head telomer (**1**).

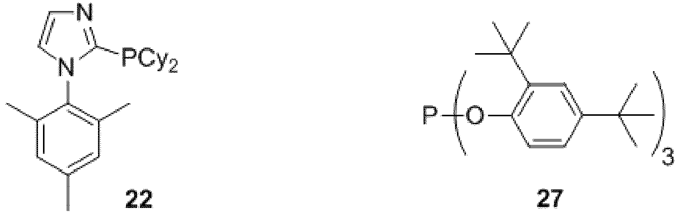

### Dicyclohexyl-[1-(2,4,6-Tris(2,4-di-tert-butylphenyl) phosphite trimethylphenyl)imidazol-2-yl]phosphane

Moreover, two catalytic systems, *i.e.,* (a) [Pd(C₃H₅)COD]BF₄/**22** in the presence of TFE and (b) Pd(OAc)₂/PPh₃ in TFE, were key in providing excellent yields and selectivities of the head-to-head telomer (**2**). In addition, [Pd(C₃H₅)COD]BF₄/**27** in the presence of ethereal solvents like Et₂O and THF, or toluene were found to provide excellent yields and selectivities of the tail-to-tail telomer (**3**). Furthermore, telomerization of dimethylbutadiene, a symmetric diene, was successfully performed using the catalytic system Pd(OAc)₂/PPh₃ (triphenylphosphine). Accordingly, the present invention provides a method for regioselective synthesis of tail-to-head, head-to-head, and tail-to-tail telomers from dienes by polymerizing a diene in the presence of specific combinations of [Pd(C₃H₅)COD]BF₄/**22**, Pd(OAc)₂/PPh₃, or [Pd(C₃H₅)COD]BF₄/**27** and polar protic solvents like MeOH (methanol), polar aprotic solvents like Et₂O (diethyl ether), or acidic polar protic solvents like TFE (trifluoroethanol). As used herein, "regioselective synthesis" refers to the selective production of a particular telomer.

As used herein, a diene is a hydrocarbon containing two carbon double bonds that may or may not be adjacent to each other. In some embodiments, the diene used in the accordance with this invention is a conjugated diene, *i.e.,* a molecule having double bonds separated by one single bond. In other embodiment, the diene is an unconjugated diene, *i.e.,* a compound having double bonds separated by two or more single bonds. Conjugated and unconjugated dienes of this invention can contain from 4 to 15 carbon atoms per molecule. Conjugated dienes containing between 4 to 15 carbon atoms per molecule include, but are not limited to, isoprene, chloroprene, 2-methoxy-1,3-butadiene, 1,3-butadiene, 1,3-pentadiene, 2,3-dimethyl-1,3-butadiene, 1,3-hexadiene, 7-methyl-3-methylene-1,6-octadiene (myrcene), farnesene, and the like. If desired, mixtures of two or more dienes can be employed in the instant methods.

In a certain embodiment, the conjugated diene has the structure of Formula I: 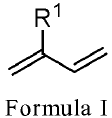 wherein R¹ is a hydrogen, halo or C₁-C₁₁ alkyl, alkoxy, alkylene, aryl, cycloalkane or alkenyl group. As used herein, "halo" refers to F, Cl, Br, or I.

"Alkyl" means a linear saturated monovalent hydrocarbon radical of one to six carbon atoms or a branched saturated monovalent hydrocarbon radical of three to six carbon atoms, e.g., methyl, ethyl, propyl, 2-propyl, butyl (including all isomeric forms), or pentyl (including all isomeric forms), and the like.

"Alkoxy" means a radical -OR where R is alkyl as defined herein, e.g., methoxy, ethoxy, propoxy, or 2-propoxy, n-, iso-, or tert-butoxy, and the like.

"Alkylene" means a linear saturated divalent hydrocarbon radical of one to six carbon atoms or as otherwise indicated or a branched saturated divalent hydrocarbon radical of two to six carbon atoms or as otherwise indicated, *e.g.,* methylene, prop-2,2-diyl, eth-1,2-diyl, prop-1,3-diyl, 1-methylprop-1,3-diyl, 2-methylprop-1,3-diyl, but-1,4-diyl (including all isomeric forms), or pent-1,5-diyl (including all isomeric forms), and the like.

"Aryl" means a monovalent, monocyclic or fused bicyclic hydrocarbon radical of 6 to 12 ring atoms, wherein the ring comprising a monocyclic radical ring is aromatic and wherein at least one of the fused rings comprising a bicyclic radical is aromatic. Unless otherwise stated, the valency of the group may be located on any atom of any ring within the radical, valency rules permitting. More specifically the term aryl includes, but is not limited to, phenyl, naphthyl, indanyl (including, for example, indan-5-yl, or indan-2-yl, and the like) or tetrahydronaphthyl (including, for example, tetrahydronaphth-1-yl, tetrahydronaphth-2-yl, and the like), and the like.

"Cycloalkane" refers to saturated cyclic hydrocarbons having from 3 to about 10 carbon atoms, more usually from about 5 to about 8 carbon atoms. Non- limiting examples of cycloalkanes include cyclopentane, cyclohexane, cycloheptane, and cyclooctane.

"Alkenyl" means a linear monovalent hydrocarbon radical of two to six carbon atoms or a branched monovalent hydrocarbon radical of three to six carbon atoms containing one or two double bonds, *e.g.,* ethenyl, propenyl (including all isomeric forms), 1-methylpropenyl, butenyl (including all isomeric forms), or pentenyl (including all isomeric forms) and the like.

Generally, the active catalytic system and method of this invention includes the use of an active catalyst precursor, a ligand, a nucleophile, a solvent and other optional additives. The active catalytic system of the present invention provides effective production of a particular telomer. As used herein, "ligand" refers to a component required to promote the formation of the active catalyst from the active catalyst precursor, *in-situ.* As used herein, an "active catalyst precursor" refers to an organometallic complex of Palladium and a stabilizing or coordinating ligand. An active catalyst precursor can be used in combination with an additional additive or ligand to generate an active catalyst *in-situ.* As used herein, "active catalyst" refers to the resulting combination of an active catalyst precursor, an additive and/or a ligand, required for making the reaction greater in effectiveness towards the production of a particular telomer.

As indicated, the catalytic system of this invention uses either [Pd(C₃H₅)(COD)]BF₄/**22**, Pd(OAc)₂/PPh₃, or [Pd(C₃H₅)(COD)]BF₄/**27** as the precursor and ligand combination. In one embodiment, [Pd(C₃H₅)(COD)]BF₄/**22** is used in the synthesis of a tail-to-head or head-to-head telomer. In another embodiment, Pd(OAc)₂/PPh₃ is used in the synthesis of a head-to-head telomer. In another embodiment, [Pd(C₃H₅)(COD)]BF₄/**27** is used in the synthesis of the tail-to-tail telomer. Selectivity for a particular telomer can be achieved when the molar ratio of precursor to ligand is between 1:3 and 1:0.1, or more preferably between 1:2 and 1:0.5, or most preferably 1:1.5 to 1:1. Similarly, precursor loading in the present method can be in the range of 0.5 to 5 mol%, or more preferably in the range of 0.5 to 3 mol%, or most preferably in the range of 0.5 to 1 mol%.

In certain embodiments, solvents like TFE, MeOH, Et₂O or a combination thereof are usedin the present method. In particular embodiments, the combination of [Pd(C₃H₅)COD]BF₄/**22** and MeOH is selective for a tail-to-head telomer, whereas the combination of [Pd(C₃H₅)COD]BF₄/**22** and TFE or Pd(OAc)₂/PPh₃ in TFE is selective for a head-to-head telomer, and the combination of [Pd(C₃H₅)(COD)]BF₄/**27** and Et₂O is selective for a tail-to-tail telomer.

The method of the invention can be carried out in the presence of a compound having at least one active hydrogen atom, *i.e.,* a nucleophile such as an amine, alcohol, water or carbanion precursor. In particular embodiments, the nucleophile is a primary or secondary amine. Examples of amines of use in the method of the invention include, but are not limited to, *n*-butylamine, dimethyl amine, and diethyl amine as well as the amines presented in Table 12. In certain embodiments the molar ratio of diene to nucleophile is in the range of about between 1:3 and 1:0.1, or more preferably between 1:2 and 1:0.5, or most preferably 1:1.5 to 1:0.5.

The telomerization reaction of this invention can be carried out continuously, semi-batch or batch-wise. Moreover, depending on the nature of the starting material, the reaction can be carried out at a temperature between -20 and 180°C. Preferably, the temperature is in the range from 20 to 150°C. More particularly, the temperature is in the range from 20 to 70°C.

As is known in the art, telomerization is a chemical reaction that creates short chain polymers, called oligomers, composed of between two to ten repeating units. In some embodiments, the method of the invention results in production of a dimer, trimer, tetramer, pentamer, hexamer, heptamer or octamer. In one embodiment, the monomeric units of the oligomer are arranged in a tail-to-head orientation. In another embodiment, the monomeric units of the oligomer are arranged in a head-to-head orientation. In another embodiment, the monomeric units of the oligomer are arranged in a tail-to-tail orientation. In certain embodiments, greater than 60%, 70%, 80%, or 90% regioselectivity is achieved using the method of this invention. Furthermore, yields using the method of the invention can be in the range of 50-100%, 60-100%, 70-100%, 80-100% or 90-100%.

Telomers synthesized in accordance with the method of the invention can be linear, branched or trienes. By way of illustration, the products presented in Table 1 can be obtained by telomerization of a compound of Formula I in accordance with the method of this invention.

**TABLE 1**

| | | | |
|---|---|---|---|
| 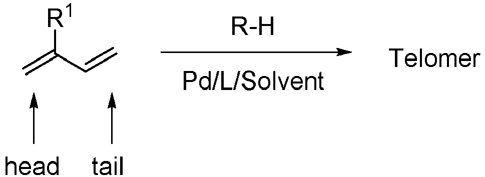 | | | |

| Telomer | Linkage | | |
|---|---|---|---|
| | Tail-To-Head | Head- To-Head | Tail-to-tail |
| Linear | 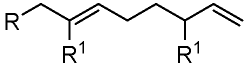 | 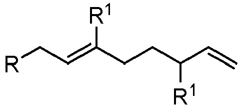 | 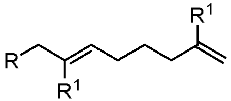 |
| Branched | 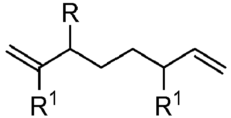 | 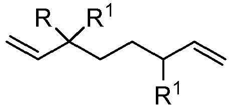 | 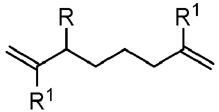 |
| Triene | 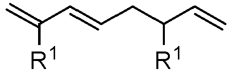 | 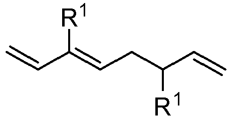 | 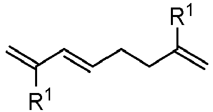 |

| | | | |
|---|---|---|---|
| R-H is a compound containing an active hydrogen. Pd = precursor, L = ligand. | | | |

The telomerization products prepared can be recovered via fractionation, distillation and/or crystallization, and may advantageously be employed as such or used for example for the synthesis of polymers, synthetic resins, surface-active agents, or non-naturally occurring terpene derivatives. Further, the diene adducts of the invention can serve as inexpensive intermediates and captive products for use in home and personal care products. Uses of these telomer terpene-like materials could also be envisioned as intermediates or captive products in markets related to uses of, or adjacent to, natural terpenes such as citronellal, citronellol, geraniol, menthol, myrcene, and other related products. Additionally, diene adducts of this invention could be useful in mint oil, confectionary, cough and cold, tobacco, oral care, and nasal care.

### Example 1: Experimental

Commercially available reagents were used as received without further purification. MeOH, NHEt₂, and isoprene were freshly distilled under N₂ according to reported procedures (Armarego & Chan (2009) Purification of Laboratory Chemicals, Elsevier). ¹H, ¹³C{¹H}, COSY, HSQC, HMBC and ³¹P{1H} NMR spectra were recorded on VARIAN MERCURY-400 and VARIAN-400-MR spectrometers. ¹H and ¹³C NMR chemical shifts were reported in parts per million (ppm) relative to CDCl₃. The GC/MS analysis was carried out on an Agilent 7890A with a MS 5975C detector using a Column HP5-MS (30 m, 0.25 mm, 0.25 µm). Product ratios were obtained by direct analysis of isolated product by GC/MS using the following conditions: 50°C for 1 minute, 50-100°C, 3°C/minute, 100-240°C, 20°C/minute.

*General Procedure for Isoprene Telomerization with Diethylamine.* In a 5 ml flask, the palladium precursor, phosphine ligand, isoprene, diethylamine and solvent, were stirred for 24 hours at room temperature. The resulting solution was evaporated under vacuum, and then distilled *in vacuo* providing the telomer products. Selectivity was determined by GC/MS.

*Synthesis of Telomer **1.*** [Pd(C₃H₅)(COD)]BF₄ (0.05 mmol), **22** (0.05 mmol), isoprene (10 mmol), diethylamine (9.6 mmol) and MeOH (2 ml), were stirred for 24 hours at room temperature. The resulting solution was handled in accordance with the general procedure providing telomer **1** as a colorless liquid in 85% yield and a 92/0/4/4 (**1/2/3/4**) ratio. ¹H NMR (CDCl₃, 400MHz) δ= 5.678 (ddd, 1H, ³*J*_{2, 1}= 17.6 Hz, ³*J*_{2, 1'}= 10.4 Hz, ³*J*_{2, 3}= 7.6 Hz, H-2); 5.254 (tq, 1H, ³*J*_{6, 5}= 7.2 Hz, ³*J*_{6, 9}= 1.2 Hz, H-6); 4.930 (br d, 1H, ³*J*_{1, 2}= 17.2 Hz, H-1); 4.896 (br d, 1H, ³*J*_{1', 2}= 10.4 Hz, H-1'); 2.855 (s, 2H, H-8); 2,431 (q, 4H, ³*J*_{11*,* 12}= 7.2 Hz, H-11); 2.103 (m, 1H, H-3); 1.995 (m, 2H, H-5); 1.606 (s, 3H, Me₍₉₎); 1.319 (m, 2H, H-4); 0.981 (t, 6H, ³*J*_{12, 11}= 7.2 Hz, H-12); 0.959 (d, 3H, ³*J*_{Me10, 3}= 6.8 Hz, Me₍₁₀₎). ¹³C NMR (CDCl₃, 400 MHz) δ= 144.880 (C-2); 133.744 (C-7); 127.524 (C-6); 112.743 (C-1); 62.366 (C-8); 46.840 (C-11); 37.635 (C-3); 36.759 (C-4); 25.747 (C-5); 20.349 (C-10); 15.262 (C-9); 11.888 (C-12).

*Synthesis of Telomer **2.*** Pd(OAc)₂ (0.05 mmol), PPh₃ (0.075 mmol), isoprene (10 mmol), diethylamine (9.6 mmol) and TFE (2 ml), were stirred for 24 hours at room temperature. The resulting solution was handled in accordance with the general procedure providing telomer **2** as a colorless liquid in an 86% yield and a 9/91/0/0 (**1/2/3/4**) ratio. ¹H NMR (CDCl₃, 400MHz) δ= 5.662 (ddd, 1H, ³*J*_{2, 1}= 17.2 Hz, ³*J*_{2, 1'}= 10 Hz, ³*J*_{2, 3}= 7.6 Hz, H-2); 5.233 (br t, 1H, ³*J*_{7, 8}= 6.8 Hz, H-7); 4.926 (br d, 1H, ³*J*_{1, 2}= 17.0 Hz, H-1); 4.885 (br d, 1H, ³*J*_{1', 2}= 10.4 Hz, H-1'); 3.038 (d, 2H, ³*J*_{8, 7}= 6.8 Hz, H-8); 2,482 (q, 4H, ³*J*_{1, 12}= 7.2 Hz, H-11); 2.055 (m, 1H, H-3); 1.959 (m, 2H, H-5); 1.604 (s, 3H, H-9); 1.364 (m, 2H, H-4); 0.995 (t, 6H, ³*J*_{12, 11}= 7.2 Hz, H-12); 0.953 (d, 3H, ³*J*_{10, 3}= 6.8 Hz, H-10). ¹³C NMR (CDCl₃, 400 MHz) δ= 144.828 (C-2); 138.128 (C-6); 121.687 (C-7); 112.753 (C-1); 50.689 (C-8); 46.858 (C-11); 37.591 (C-3); 37.568 (C-5); 34.954 (C-4); 20.313 (C-10); 16.544 (C-9); 11.999 (C-12).

*Synthesis of Telomer **3.*** [Pd(C₃H₅)COD]BF₄ (0.05 mmol), **27** (0.075 mmol), isoprene (10 mmol), diethylamine (9.6 mmol), Et₂O (2 ml), were stirred for 24h at 40°C. The resulting solution handled in accordance with the general procedure providing telomer **3** as a colorless liquid in 90% yield and a 4/0/90/6 (**1/2/3/4**) ratio. ¹H NMR (CDCl₃, 400MHz) δ= 5.299 (tq, 1H, ³*J*_{6,5}= 7.2 Hz, ⁴*J*_{6,9}= 1.2Hz, H-6); 4.702 (br s, 1H, H-1/H-1'); 4.673 (br s, 1H, H-1/H-1'); 2.884 (s, 2H, H-8); 2.453 (q, 4H, ³*J*_{11,12}= 7.2 Hz, H-1); 2,020 (m, 4H, H-5 and H-4/H-3); 1.717 (s, 3H, Me(10)); 1.634 (s, 3H, Me(9)); 1.514 (m, 2H, H-3/H-4); 0.995 (t, 6H, ³*J*_{12,11}= 7.2 Hz, H-11). ¹³C NMR (CDCl₃, 400 MHz) δ= 146.218 (C-2); 133.988 (C-7); 127.367 (C-6); 109.931 (C-1); 62.363 (C-8); 46.640 (C-11); 37.603 (C-3/C-4); 27.864 and 27.598 (C-5 and C4/C3); 22.597 (C-10); 15.275 (C-9); 11.677 (C-12).

### Example 2: Telomerization of Isoprene

A screening of various Pd precursors and ligands was first performed to evaluate their influence on the catalytic output of isoprene telomerization. The results using monophosphine ligands are summarized in Table 2.

The telomerization of isoprene with Pd/PPh₃ and Pd/**10** has been previously reported. Aiming to know the effect of the precursor under similar reaction conditions, Pd(OAc)₂, PdCl₂ or [Pd(C₃H₅)COD]BF₄ (COD=cyclooctadiene) were tested in the presence of PPh₃ obtaining in all cases full conversion and a similar mixture of linear telomers (Table 2, Entries 1-2), with a slight increase in selectivity for telomer **1** when [Pd(C₃H₅)COD]BF₄ was used (entry 3).

**TABLE 2**

| | | | |
|---|---|---|---|
| 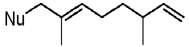 | 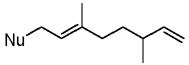 | 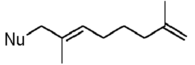 | 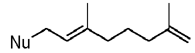 |
| **1** | **2** | **3** | **4** |

| Entry^{a} | Ligand | Yield Telomers (%) | Selectivity (**1/2/3/4**) |
|---|---|---|---|
| 1 | PPh₃ | >99 | 51/29/19/1 |
| 2^{b} | PPh₃ | >99 | 55/12/25/8 |
| 3^{c} | PPh₃ | >99 | 70/8/22/0 |
| 4^{d} | PPh₃ | 53 | 51/27/20/2 |
| 5 | P[2,4,6(OMe)₃-C₆H₂]₃ | 2 | 6/94/0/0 |
| 6^{e} | P[2,4,6(OMe)₃-C₆H₂]₃ | 19 | 5/90/5/0 |
| 7^{c} | P[2,4,6(OMe)₃-C₆H₂]₃ | 83 | 5/94/1/0 |
| 8 | P(2-OMe-C₆H₄)₃ | 73 | 52/38/10/0 |
| 9 | P(3,5-CF₃-C₆H₃)₃ | 0 | |
| 10 | P(4-CF₃-C₆H₄)₃ | 4 | 55/0/45/0 |
| 11 | P(C₆F₅)₃ | 7 | 70/11/15/4 |

| | | | |
|---|---|---|---|
| ^{a}Reaction conditions: Pd(OAc)₂ (0.05 mmol), Ligand (0.075 mmol), isoprene (10 mmol), NHEt₂ (9.6 mmol), MeOH (2 ml), room temperature, 24 hours. ^{b}PdCl₂ (0.05 mmol) used instead of Pd(OAc)₂. ^{c}[Pd(C₃H₅)COD]BF₄ (0.05 mmol) used instead of Pd(OAc)₂. ^{d}50 µl of H₂O used as additive. ^{e}48 hours. | | | |

As PdCl₂ was poorly soluble in MeOH, Pd(OAc)₂ was selected as precursor for subsequent experiments. Upon addition of water to the reaction mixture, the yield decreased, but the selectivity remained unchanged (Entry 4, compared with Entry 1).

The catalytic systems bearing the electron rich phosphine P[2,4,6(OMe)₃-C₆H₂]₃ (**10**) afforded high selectivity toward telomer **2.** However, the yields strongly depended on the palladium precursor used, achieving up to 83% yield and 94% selectivity when [Pd(C₃H₅)COD]BF₄ was used (Entries 5-7). The tris(di-*o*-methoxyphenyl)phosphine ligand, which is a basic phosphine with less steric hindrance than ligand **10,** was also tested (Entry 8) but provided lower selectivity. The use of fluorinated ligands provided very low conversion with no relevant selectivities (Entries 9-11).

The following monophosphines and diphosphines were also tested and the results are summarized in Tables 3-4, and 10.

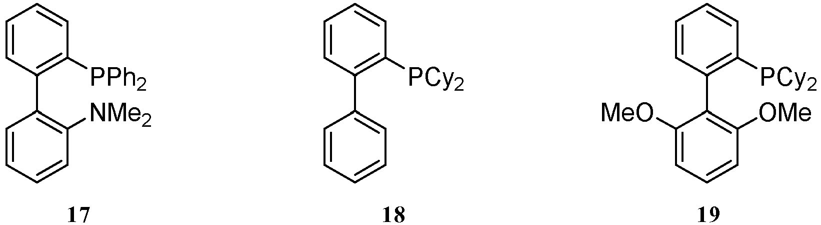

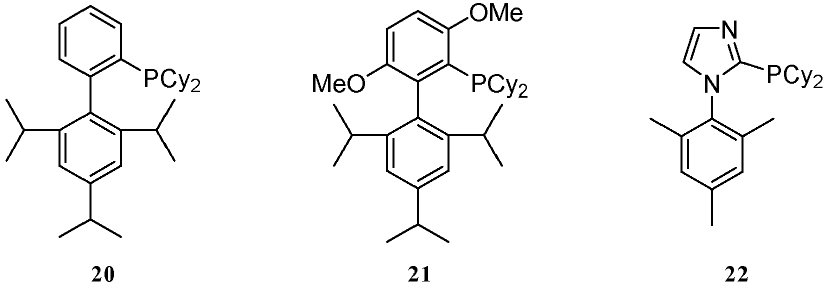

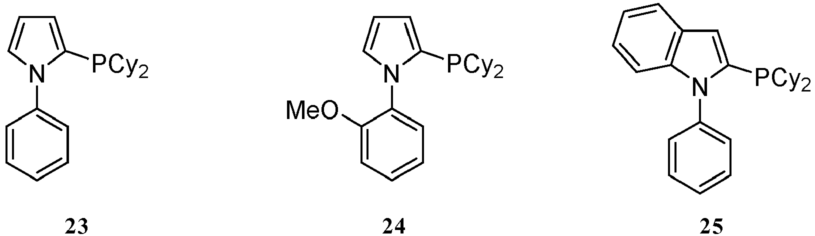

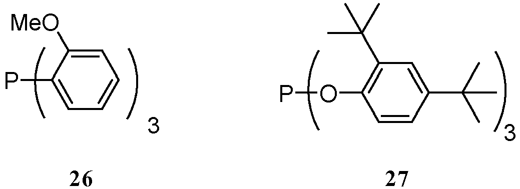

**TABLE 3**

| | | | |
|---|---|---|---|
| 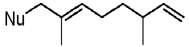 | 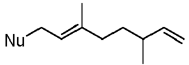 | 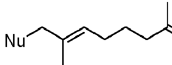 | 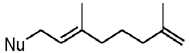 |
| **1** | **2** | **3** | **4** |

| Entry^{a} | Ligand | Yield Telomers (%) | Selectivity (**1/2/3/4**) |
|---|---|---|---|
| 1^{b} | **17** | 78 | 70/7/20/3 |
| 2 | **18** | 93 | 75/8/15/2 |
| 3 | **19** | 90 | 76/9/14/1 |
| 4^{b} | **20** | 92 | 73/6/21/0 |
| 5 | **21** | 0^{C} | - |
| 6 | **22** | 11 | 83/3/10/4 |
| 7^{d} | **22** | 88 | 90/0/6/4 |
| 8^{d} | **23** | 100 | 67/33/0/0 |
| 9^{d} | **23** | 100 | 60/40/0/0 |
| 10^{d} | **25** | 80 | 75/25/0/0 |

| | | | |
|---|---|---|---|
| ^{a}Reaction conditions: Pd(OAc)₂ (0.05 mmol), L (0.075 mmol), isoprene (10 mmol), NHEt₂ (9.6 mmol), MeOH (2 ml), room temperature, 24 hours. ^{b}Pd/L ratio = 1/1. ^{c}Hydroamination products were only formed (29% yield monomers). ^{d}[Pd(C₃H₅)COD]BF₄ (0.05 mmol) used instead of Pd(OAc)₂. | | | |

Phosphines bearing a biphenyl or phenyl-azol moiety are very successful ligands in various transition metal catalyzed processes. Phosphines **17, 18, 19** and **20** provided close to 90% yield with moderate selectivity to telomer **1** (Entries 1-4). However, the more sterically hindered phosphine **21** did not provide telomerization products and only hydroamination products were recovered (Entry 5). Phosphorus ligands bearing pyrrole, imidazole or indole moieties were also tested (Entries 6-10). The catalytic system bearing ligand **22** afforded low yield and high selectivity to telomer **1** (Entry 6). Both yield and selectivity were improved when [Pd(C₃H₅)COD]BF₄ was used as precursor, achieving 88% yield and 90% selectivity (Entry 7). The catalysts bearing phosphines bearing a pyrrole group (**23-25**) provided excellent conversions, but selectivity for telomer **1** decreased (Entries 8-10).

The influence of diphosphine ligands with different bite angles was also evaluated in this reaction. The results are summarized in Table 4.

**TABLE 4**

| | | | |
|---|---|---|---|
| 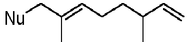 | 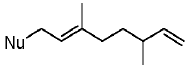 | 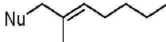 | 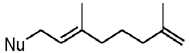 |
| **1** | **2** | **3** | **4** |

| Entry^{a} | Ligand | Yield Telomers(%) | Selectivity (**1/2/3/4**) |
|---|---|---|---|
| 1 | dppm | 26 | 71/14/14/1 |
| 2 | dppe | 59 | 76/5/19/0 |
| 3 | dppb | 69 | 71/10/19/0 |
| 4 | dppp | 69 | 69/12/19/0 |
| 5 | BINAP | 0 | - |
| 6 | DPEphos | 0^{b} | - |
| 7 | XANTphos | 5 | 52/24/24/0 |
| 8 | XANTphos(NEt)₂ | 25 | 66/13/18/3 |

| | | | |
|---|---|---|---|
| ^{a}Reaction conditions: [Pd(C₃H₅)COD]BF₄ (0.05 mmol), ligand (0.05 mmol), isoprene (10 mmol), NHEt₂ (9.6 mmol), MeOH (2 ml), room temperature, 24 hours. ^{b}Hydroamination products were only formed (45% yield monomers). | | | |

Initial tests with diphosphines dppm, dppe, dppb and dppp showed that the most relevant results were obtained using [Pd(C₃H₅)COD]BF₄ as precursor, rather than Pd(OAc)₂ or PdCl₂. These ligands provided similar results in terms of selectivity, despite their difference in bite angles (Table 4, Entries 1-4). The systems bearing diphosphines BINAP, DPEphos or XANTphos were not active in the telomerization of isoprene (Entries 5-7), and only the bis-diethylamino derivative of XANTphos provide an active system, although low yield was obtained (Entry 8). Similar to the results obtained with the bulky monophosphine **21** the products of hydroamination were formed using DPEphos. It could be concluded that the use of bulky ligands did not favour the coordination of two molecules of isoprene, and consequently favoured the hydroamination of the substrate under these conditions.

From this analysis, it was concluded that [Pd(C₃H₅)COD]BF₄/**22** was an excellent catalytic system for the telomerization of isoprene providing high yields and selectivities for the telomer **1.** In this context, the Pd/L ratio, Pd loading, solvent, and temperature (Tables 5-7) were analyzed.

**TABLE 5**

| | | | | |
|---|---|---|---|---|
| 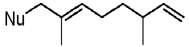 | | 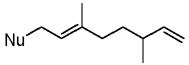 | 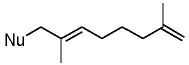 | 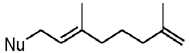 |
| **1** | | **2** | **3** | **4** |

| Entry^{a} | Pd/L ratio | Pd loading | Yield Telomers (%) | Selectivity (**1/2/3/4**) |
|---|---|---|---|---|
| 1 | 1/2 | 0.5 | 69 | 90/0/6/4 |
| 2 | 1/1.5 | 0.5 | 88 | 90/0/6/4 |
| 3 | 1/1 | 0.5 | 85 | 90/0/6/4 |
| 4 | 1/0.5 | 0.5 | 77 | 90/0/7/3 |
| 5 | 1/1 | 1 | 89 | 67/20/13/0 |
| 6 | 1/1 | 3 | 95 | 60/25/15/0 |

| | | | | |
|---|---|---|---|---|
| ^{a}Reaction conditions: [Pd(C₃H₅)COD]BF₄, **22,** isoprene (10 mmol), NHEt₂ (9.6 mmol), MeOH (2 ml), 24 hours, room temperature. | | | | |

This analysis indicated that when the Pd/L ratio was varied, yield was slightly altered but the selectivity remained practically unchanged (Entries 1-4). Noteworthy, the use of a 1/1 ratio provided 85% yield and 90% selectivity to telomer **1** (Entry 3). When 1 or 3 mol% of Pd were used (Entries 5 and 6), the yield reached 95% but the selectivity to **1** decreased to *ca.* 60% under these conditions. Different palladium species could be formed at different concentrations.

The effect of various protic and aprotic solvents was also evaluated (Table 6).

**TABLE 6**

| | | | |
|---|---|---|---|
| 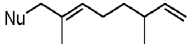 | 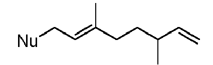 | 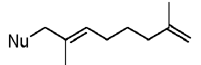 | 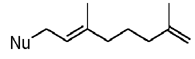 |
| **1** | **2** | **3** | **4** |

| Entry^{a} | Solvent | Yield Telomers (%) | Selectivity (**1/2/3/4**) |
|---|---|---|---|
| 1 | EtOH | 81 | 90/0/7/3 |
| 2 | PrOH | 94 | 88/0/9/3 |
| 3 | ⁱPrOH | 79 | 87/0/10/3 |
| 4 | Toluene | 65 | 90/0/9/1 |
| 5 | Acetone | 65 | 85/0/11/4 |
| 6 | THF | 58 | 85/0/14/1 |
| 7 | ACN | 67 | 69/15/13/3 |
| 8 | Hexane | 5 | 36/11/52/1 |
| 9 | TFE | 86 | 9/91/0/0 |

| | | | |
|---|---|---|---|
| ^{a}Reaction conditions: [Pd(C₃H₅)COD]BF₄ (0.05 mmol), **22** (0.05 mmol), isoprene (10 mmol), NHEt₂ (9.6 mmol), solvent (2 ml), 24 hours, room temperature. ^{b}Pd(OAc)₂ (0.05 mmol) used instead of [Pd(C₃H₅)COD]BF₄ | | | |

The use of EtOH, PrOH and ⁱPrOH provided high yields (>79%) and selectivities to **1** (>87%) (Entries 1-3). When toluene, acetone and THF were tested, high selectivities to **1** were also obtained, but yields slightly decrease (Entries 4-6). While acetonitrile provided moderate yield and selectivities to telomer **1** (Entry 7), low yield and moderate selectivity to telomer **3** were obtained by hexane (Entry 8). Furthermore, the use of TFE provided a striking selectivity to telomer **2** (91%) in high yield (Entry 9). This latter system was further analyzed and the results are described in Tables 8 and 9.

The influence of the isoprene/NHEt₂ molar ratio on the catalytic performance of this system was studied (Table 7).

**TABLE 7**

| | | | | |
|---|---|---|---|---|
| 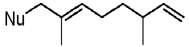 | | 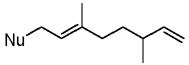 | 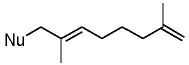 | 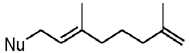 |
| **1** | | **2** | **3** | **4** |

| Entry^{a} | Isoprene/NHEt₂ Ratio | Molar Temperature (°C) | Yield Telomers (%) | Selectivity (**1/2/3/4**) |
|---|---|---|---|---|
| 1 | 1/1.37 | r.t. | 34 | 78/12/10/0 |
| 2 | 1/2.74 | r.t. | 25 | 77/10/13/0 |
| 3^{b} | 1/0.96 | -10 | 19 | 94/0/4/2 |
| 4 | 1/0.96 | 40 | 91 | 68/16/16/0 |

| | | | | |
|---|---|---|---|---|
| ^{a}Reaction conditions: [Pd(C₃H₅)COD]BF₄ (0.05 mmol), **22** (0.05 mmol), isoprene (10 mmol), NHEt₂, MeOH (2 ml), 24 hours. ^{b}72 hours | | | | |

Increasing the concentration of NHEt₂ resulted in a drop in both yield and selectivity of telomer **1** (Entries 1 and 2). Further, when the reaction was carried out at -10°C, low yield (19%) and high selectivity (94%) were achieved (Entry 3). However, at 40°C, the selectivity for telomer **1** decreased to 68% (Entry 4).

In the previous screening of solvents it was found that when the reaction was driven in trifluoroethanol, telomer **2** was obtained in excellent yields and selectivities. Thus, other parameters of the reaction were studied. Initially, the effect of the Pd loading was evaluated, the results of which are presented in Table 8.

**TABLE 8**

| | | | |
|---|---|---|---|
| 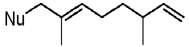 | 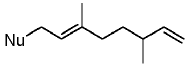 | 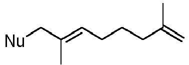 | 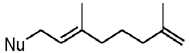 |
| **1** | **2** | **3** | **4** |

| Entry^{a} | Pd loading (mol%) | Yield Telomers (%) | Selectivity (**1/2/3/4**) |
|---|---|---|---|
| 1 | 0.5 | 46 | 7/93/0/0 |
| 2 | 1 | 66 | 9/91/0/0 |
| 3 | 3 | 86 | 3/97/0/0 |

| | | | |
|---|---|---|---|
| ^{a}Reaction conditions: [Pd(C₃H₅)COD]BF₄ (0.1 mmol), **22** (0.1 mmol), isoprene (10 mmol), NHEt₂ (9.6 mmol), TFE (2 ml), 24 hours, room temperature. | | | |

The increase in Pd precursor loading revealed a significant influence on both yield and selectivity (Entries 1-3). Increasing catalyst precursor from 0.5% to 3% significantly increased yields of telomer products to 86% (Entry 3). Selectivity also increased, providing 97% of telomer **2** (Entry 3).

When the amount of NHEt₂ was increased (Table 9), the selectivity toward telomer **2** decreased, favoring the formation of telomer **1** up to 67% when 2.74 eq. of NHEt₂ were used (Entries 1 and 2).

**TABLE 9**

| Entry^{a} | Isoprene/NHEt₂ Molar Ratio | Yield Telomers (%) | Selectivity (**1/2/3/4**) |
|---|---|---|---|
| 1 | 1/1.37 | 59 | 26/74/0/0 |
| 2 | 1/2.74 | 91 | 67/29/4/0 |

| | | | |
|---|---|---|---|
| ^{a}Reaction conditions: [Pd(C₃H₅)COD]BF₄ (0.1 mmol), **22** (0.1 mmol), isoprene (10 mmol), NHEt₂, TFE (2 ml), 24 hours, room temperature. | | | |

The behavior of other catalytic systems such as Pd(OAc)₂/PPh₃ in TFE was also determined. Using this catalytic system, 86% yield and 91% selectivity toward telomer **2** was achieved. Interestingly, conversion and selectivity were similar to that obtained with Pd/**22** as the catalytic system, which indicated that under these reaction conditions, the selectivity was determined by the solvent.

The first selective system affording telomer **3** that was identified was Pd(OAc)₂/PPh₃ in the presence of DMF and different amounts of NEt₃, which provided good yields (63%-74%) and selectivity (81% -87%) (Table 10, Entries 1 and 2). Using the conditions that provided highest selectivity to **3,** that is using 20 mmols of NEt₃ as additive, different ligands were tested. The use of **26,** provided low yield (29%) and high selectivity to **3** (84%) (Entry 3). Moderate yields (36%-58%) and high selectivity to **3** was obtained using dppp (88%) and dppb (91%) (Entries 4 and 5). The catalytic system [Pd(C₃H₅)COD]BF₄/**27** in presence of DMF provided moderate yield (61%) and high selectivity to **3** (89%) (Entry 6). Yield was increased to 81% using THF instead of DMF with a selectivity up to 87% to **3** (Entry 7), and improved further using the same catalyst by replacing THF with Et₂O at slightly higher reaction temperature (Entry 10).

**TABLE 10**

| Entry^{a} | Pd/L system | Solvent | Yield Telomers (%) | Selectivity (**1/2/3/4**) |
|---|---|---|---|---|
| 1^{b,e} | Pd(OAc)₂/PPh₃ | DMF/NEt₃ | 74 | 3/10/81/6 |
| 2^{e} | Pd(OAc)₂/PPh₃ | DMF | 63 | 2/8/87/3 |
| 3^{e} | Pd(OAc)₂/**26** | DMF | 29 | 7/9/84/0 |
| 4^{f} | Pd(OAc)₂/dppp | DMF | 36 | 3/5/88/4 |
| 5^{f} | Pd(OAc)₂/dppb | DMF | 58 | 2/5/91/2 |
| 6^{e} | [Pd(C₃H₅)COD]BF₄/**27** | DMF | 61 | 4/2/89/5 |
| 7^{c,e} | [Pd(C₃H₅)COD]BF₄/**27** | THF | 81 | 6/0/87/7 |
| 8^{d,e} | [Pd(C₃H₅)COD]BF₄/**27** | Hexane | 41 | 2/2/94/2 |
| 9^{d,e} | [Pd(C₃H₅)COD]BF₄/**27** | Toluene | 75 | 4/0/94/2 |
| 10^{d,e} | [Pd(C₃H₅)COD]BF₄/**27** | Et₂O | 90 | 4/0/90/6 |

| | | | | |
|---|---|---|---|---|
| ^{a}Conditions: [Pd] (0.05 mmol), L, isoprene (10 mmol), NHEt₂ (9.6 mmol), solvent (2ml), 80°C, 24h, sealed tube. ^{b}NEt₃ (10 mmols). ^{c}Room temperature. ^{d}Reaction temperature: 40°C. ^{e}Pd/L ratio: 1/1.5. ^{f}Pd/L ratio: 1/1. | | | | |

Efforts to improve upon the highest yield/selectivity ratio using [Pd(C₃H₅)COD]BF₄/**27** was further explored by evaluating the effect of the [Pd] precursor, [Pd] loading, the use of Et₃N as additive, temperatures and different solvents (Table 11). The best results remained to be [Pd(C₃H₅)COD]BF₄/**27** in the presence of Et₂O. Therefore, it has been found that Et₃N is not a necessary additive when [Pd(C₃H₅)COD]BF₄ is used in combination with ligand **27** and ethereal solvents like THF or Et₂O.

**TABLE 11**

| Entry^{a} | Pd mol% | Solvents | Yield Telomeres | Selectivity (**1/2/3/4**) |
|---|---|---|---|---|
| 1^{d} | 0,5 | THF | 81 | 6/0/87/7 |
| 2^{b,d} | 0.5 | THF | 0 | - |
| 3^{b,e} | 0.5 | THF | 0 | - |
| 4^{d} | 1 | THF | 82 | 13/0/72/15 |
| 5^{d} | 3 | THF | 72 | 23/1/53/23 |
| 6^{c,d} | 0.5 | THF | 22 | 5/0/90/5 |
| 7^{f} | 0.5 | THF | 91 | 7/0/85/8 |
| 8^{f} | 0.5 | DMF | 25 | 6/0/89/5 |
| 9^{f} | 0.5 | DCM | 85 | 32/6/27/35 |
| 10^{f} | 0,5 | Hexane | 41 | 2/2/94/2 |
| 11^{f} | 0.5 | Toluene | 75 | 4/0/94/2 |
| 12^{f} | 0.5 | Et₂O | 90 | 4/0/90/6 |

| | | | | |
|---|---|---|---|---|
| ^{a}Conditions: [Pd(C₃H₅)COD]BF₄, **27** (1.5 eq.), isoprene (10 mmol), NHEt₂ (9.6 mmol), solvent (2ml). ^{b}Pd(OAc)₂ (0.05 mmol) used as precursor. ^{c}NEt₃ (15 mmols) used as additive. ^{d}Reaction temperature: room temperature. ^{e}Reaction temperature: 70°C. ^{f}Reaction temperature: 40°C. All reactions were carried out for 24 hours. | | | | |

In order to know the influence of the nucleophile, the following amines were tested under the catalytic system that provided the best yield and selectivity towards telomer **2** using diethylamine: diisopropylamine (iPr₂NH), dibenzylamine (Bn₂NH), morpholine, and cyclopentylamine. The results of this analysis are presented in Table 12.

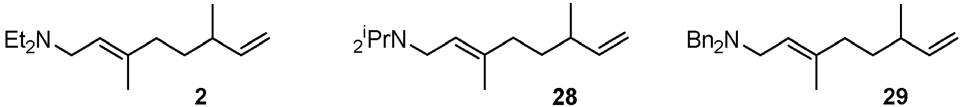

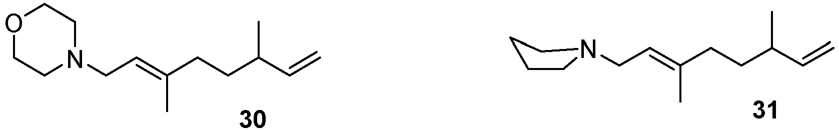

**TABLE 12**

| Amine | Yield (%) | Selectivity (%) |
|---|---|---|
| **2** | 86 | 91 |
| **28** | 5 | <90 |
| **29** | Trace | 82 |
| **30** | 67 | 89 |
| **31** | 34 | 91 |

High selectivity toward the telomers head-to-head (**28-31**) was obtained in all cases, although yield strongly depended on the hindrance of the secondary amine. Thus, Compounds **28** and **29** were obtained in very low yield using the bulky amines *i-*Pr₂NH and Bn₂NH.

Based upon the above-referenced analyses, optimal yield and selectivity of telomer **1** is achieved using [Pd(C₃H₅)COD]BF₄/**22** and HNEt₂ in combination with a variety of solvents under the conditions listed in Table 13. Similarly, optimal yield and selectivity of telomer **2** is achieved using [Pd(C₃H₅)COD]BF₄/**22** (or Pd(OAc)₂, PPh₃) and HNEt₂ in combination with TFE under the conditions listed in Table 13. Further, optimal yield and selectivity of telomer **3** is achieved using [Pd(C₃H₅)COD]BF₄/**27** and HNEt₂ in combination with ethers such as Et₂O or THF under the conditions listed in Table 13.

**TABLE 13**

| Condition | Telomer | | |
|---|---|---|---|
| | **1^{a}** | **2^{b}** | **3^{c}** |
| Pd/L ratio | 1:0.5* to 1:2^{#} | 1:0.5* to 1:2^{#} | 1:0.5* to 1:2^{#} |
| Pd loading | 0.5mol%* to 0.9mol%^{#} | 0.5mol%^{*} to 3mol%^{*} | 0.5mol%* to 0.9mol%^{#} |
| Isoprene/HNEt₂ ratio | 1:0.51^{#} to 1:1.36^{#} | 1:0.50* to 1:1.36^{#} | 1:0.51^{#} to 1:1.36^{#} |
| Temperature | -10°C* to 49°C^{#} | -10°C* to 28°C^{#} | 28°C*^{#} to 40°C* |

| | | | |
|---|---|---|---|
| ^{a}[Pd(C₃H₅)COD]BF₄/**22**/MeOH. ^{b}[Pd(C₃H₅)COD]BF₄/**22**/TFE. ^{c}[Pd(C₃H₅)COD]BF₄/**27**/Et₂O. *Yield lower. ^{#}Selectivity lower. | | | |

### Example 3: Telomerization of Other Dienes

There are very few examples of telomerization of dienes other than butadiene and isoprene (Consiglio & Waymouth (1989) *supra;* Clement, et al. (2008) *supra;* van Leeuwen, et al. (2011) *supra;* Grotevendt, et al. (2007) *supra;* Tschan, et al. (2010) *supra*). Accordingly, the instant analysis was extended to assess the telomerization of dimethylbutadiene (Scheme 3).

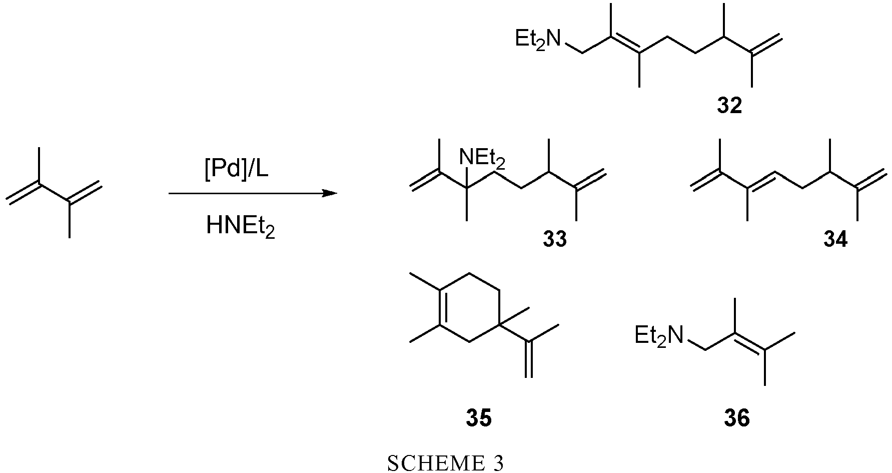

The results obtained in the telomerization of this substrate using Pd(OAc)₂/PPh₃ as a catalytic system are presented in Table 14.

**TABLE 14**

| Entry^{a} | Isoprene/NHEt₂ Molar Ratio | Temp. (°C) | Conversion (%) | Yield Telomers (%) | Selectivity (**32/33/34/35/36**) |
|---|---|---|---|---|---|
| 1 | 1/0.96 | r.t. | 0 | 0 | - |
| 2 | 1/0.96 | 70 | 72 | 53 | 84/0/0/0/16 |
| 3 | 1/1.94 | 70 | 64 | 51 | 76/0/0/0/24 |
| 4^{b} | 1/0.96 | 70 | 0 | 0 | - |
| 5^{c} | 1/0.96 | 70 | 65 | 57 | 88/0/4/1/7 |

| | | | | | |
|---|---|---|---|---|---|
| ^{a}Reaction conditions: Pd(OAc)₂ (0.05 mmol), PPh₃ (0.075 mmol), dimethylbutadiene (10 mmol), NHEt₂ MeOH (2 ml), 24 hours. ^{b}TFE (2 ml) was used instead of MeOH. ^{c}dppe (0.05 mmol). | | | | | |

When the reaction was carried at room temperature, telomerization products were not detected (Entry 1). However, upon increasing the temperature to 70°C, a mixture of telomer **32** and the monomer **36** was obtained (Entry 2). Both products were easily separated by distillation *in vacuo.* It is noteworthy that the branched product **33** was not observed either by NMR or by GC/MS techniques. When the amount of diethylamine was increased, conversion was similar, but the amount of **36** increased (Entry 3). Unexpectedly, using TFE as the solvent, no conversion was observed (Entry 4). However, the replacement of triphenylphosphine by a diphosphine (dppe) provided a similar conversion (57%) and the selectivity increased to 88%, decreasing significantly the monomer **36** (Entry 5).

## Claims

1. A method for regioselective synthesis of a telomer from isoprene comprising the step of polymerizing the isoprene in the presence of an active catalytic system containing:
(a) an active catalyst precursor and a ligand, wherein
(i) the active catalyst precursor is selected from the group consisting of [Pd(C₃H₅)COD]BF₄ and Pd(OAC)_{2;} and
(ii) the ligand is selected from the group consisting of dicyclohexyl-[1-(2,4,6-trimethylphenyl) imidazol-2-yl]phosphane, triphenylphosphine and tris(2,4-di-tert-butylphenyl)phosphite; and
(b) a solvent comprising methanol, ethanol, propanol, iso-propanol, toluene, acetone, acetonitrile, trifluoroethanol, diethyl ether or tetrahydrofuran.

2. The method of claim 1, wherein the active catalyst precursor and the ligand have a molar ratio between 1:3 and 1:0.1.

3. The method of claim 1, or claim 2, wherein the active catalyst precursor is [Pd(C₃H₅)COD]BF₄; the ligand is dicyclohexyl-[1-(2,4,6-trimethylphenyl)imidazol-2-yl]phosphane; and the solvent comprises methanol, ethanol, propanol, iso-propanol, toluene, acetone, or acetonitrile and wherein the telomer comprises a tail-to-head telomer.

4. The method of claim 3, wherein the tail-to-head telomer has a yield of greater than 60% by weight.

5. The method of claim 1, or of any preceding claim, wherein the active catalyst precursor is [Pd(C₃H₅)COD]BF₄; the ligand is triphenylphosphine; and the solvent comprises trifluoroethanol, and wherein the telomer comprises a head-to-head telomer.

6. The method of claim 5, wherein the head-to-head telomer has a yield of greater than 60% by weight.

7. The method of claim 1, or of any of claims 2 to 4, wherein the active catalyst precursor is Pd(OAc)₂; the ligand is triphenylphosphine; and the solvent comprises trifluoroethanol, and wherein the telomer comprises a head-to-head telomer.

8. The method of claim 7, wherein the head-to-head telomer has a yield of greater than 60% by weight.

9. The method of claim 1, or of any of claims 2 to 4, wherein the active catalyst precursor is [Pd(C₃H₅)COD]BF₄; the ligand is tris(2,4-di-tert-butylphenyl)phosphite; and the solvent comprises diethyl ether, tetrahydrofuran or toluene, and wherein the telomer comprises a tail-to-tail telomer.

10. The method of claim 9, wherein the tail-to-tail telomer has a yield of greater than 60% by weight.

11. The method of claim 1, or of any of the preceding claims, wherein the active catalytic system further contains a nucleophile.

12. The method of claim 11, wherein the nucleophile comprises an amine.

## Patentansprüche

1. Verfahren zur regioselektiven Synthese eines Telomers aus Isopren, umfassend den Schritt des Polymerisierens des Isoprens in Gegenwart eines aktiven katalytischen Systems, das
(a) eine Vorstufe des aktiven Katalysators und einen Liganden, wobei
(i) die Vorstufe des aktiven Katalysators aus der Gruppe bestehend aus [Pd(C₃H₅)COD]BF₄ und Pd(OAc)₂ ausgewählt ist und
(ii) der Ligand aus der Gruppe bestehend aus Dicyclohexyl[1-(2,4,6-trimethylphenyl)-imidazol-2-yl]phosphan, Triphenylphosphin und Tris(2,4-di-tert-butylphenyl)phosphit ausgewählt ist; und
(b) ein Lösungsmittel, das Methanol, Ethanol, Propanol, Isopropanol, Toluol, Aceton, Acetonitril, Trifluorethanol, Diethylether oder Tetrahydrofuran umfasst;
enthält.

2. Verfahren nach Anspruch 1, bei dem die Vorstufe des aktiven Katalysators und der Ligand ein Molverhältnis zwischen 1:3 und 1:0,1 aufweisen.

3. Verfahren nach Anspruch 1 oder Anspruch 2, bei dem es sich bei der Vorstufe des aktiven Katalysators um [Pd(C₃H₅)COD]BF₄ handelt; es sich bei dem Liganden um Dicyclohexyl[1-(2,4,6-trimethylphenyl)imidazol-2-yl]phosphan handelt und das Lösungsmittel Methanol, Ethanol, Propanol, Isopropanol, Toluol, Aceton oder Acetonitril umfasst und bei dem das Telomer ein Schwanz-Kopf-Telomer umfasst.

4. Verfahren nach Anspruch 3, bei dem das Schwanz-Kopf-Telomer eine Ausbeute von mehr als 60 Gew.-% aufweist.

5. Verfahren nach Anspruch 1 oder einem der vorhergehenden Ansprüche, bei dem es sich bei der Vorstufe des aktiven Katalysators um [Pd(C₃H₅)COD]BF₄ handelt; es sich bei dem Liganden um Triphenylphosphin handelt und das Lösungsmittel Trifluorethanol umfasst und bei dem das Telomer ein Kopf-Kopf-Telomer umfasst.

6. Verfahren nach Anspruch 5, bei dem das Kopf-Kopf-Telomer eine Ausbeute von mehr als 60 Gew.-% aufweist.

7. Verfahren nach Anspruch 1 oder einem der Ansprüche 2 bis 4, bei dem es sich bei der Vorstufe des aktiven Katalysators um Pd(OAc)₂ handelt; es sich bei dem Liganden um Triphenylphosphin handelt und das Lösungsmittel Trifluorethanol umfasst und bei dem das Telomer ein Kopf-Kopf-Telomer umfasst.

8. Verfahren nach Anspruch 7, bei dem das Kopf-Kopf-Telomer eine Ausbeute von mehr als 60 Gew.-% aufweist.

9. Verfahren nach Anspruch 1 oder einem der Ansprüche 2 bis 4, bei dem es sich bei der Vorstufe des aktiven Katalysators um [Pd(C₃H₅)COD]BF₄ handelt; es sich bei dem Liganden um Tris(2,4-di-tert-butylphenyl)phosphit handelt und das Lösungsmittel Diethylether, Tetrahydrofuran oder Toluol umfasst und bei dem das Telomer ein Schwanz-Schwanz-Telomer umfasst.

10. Verfahren nach Anspruch 9, bei dem das Schwanz-Schwanz-Telomer eine Ausbeute von mehr als 60 Gew.-% aufweist.

11. Verfahren nach Anspruch 1 oder einem der vorhergehenden Ansprüche, bei dem das aktive katalytische System ferner ein Nucleophil enthält.

12. Verfahren nach Anspruch 11, bei dem das Nucleophil ein Amin umfasst.

## Revendications

1. Procédé pour de synthèse régiosélective d'un télomère à partir d'isoprène comprenant l'étape de polymérisation de l'isoprène en présence d'un système catalytique actif contenant :
(a) un précurseur de catalyseur actif et un ligand, où
(i) le précurseur de catalyseur actif est choisi dans le groupe constitué de [Pd(C₃H₅)COD]BF₄ et Pd(OAc)₂ ; et
(ii) le ligand est choisi dans le groupe constitué des dicyclohexyl-[1-(2,4,6-triméthylphényl)imidazol-2-yl]phosphane, triphénylphosphine et tris(2,4-di-tert-butylphényl)phosphite ; et
(b) un solvant comprenant le méthanol, l'éthanol, le propanol, l'isopropanol, le toluène, l'acétone, l'acétonitrile, le trifluoroéthanol, l'éther diéthylique ou le tétrahydrofurane.

2. Procédé selon la revendication 1, dans lequel le précurseur de catalyseur actif et le ligand ont un rapport molaire compris entre 1:3 et 1:0,1.

3. Procédé selon la revendication 1, ou la revendication 2, dans lequel le précurseur de catalyseur actif est [Pd(C₃H₅)COD]BF₄ ; le ligand est le dicyclohexyl-[1-(2,4,6-triméthylphényl)imidazol-2-yl]phosphane ; et le solvant comprend le méthanol, l'éthanol, le propanol, l'isopropanol, le toluène, l'acétone ou l'acétonitrile et dans lequel le télomère comprend un télomère queue-à-tête.

4. Procédé selon la revendication 3, dans lequel le télomère queue-à-tête a un rendement supérieur à 60 % en poids.

5. Procédé selon la revendication 1, ou selon l'une quelconque des revendications précédentes, dans lequel le précurseur de catalyseur actif est [Pd(C₃H₅)COD]BF₄ ; le ligand est la triphénylphosphine ; et le solvant comprend le trifluoroéthanol, et dans lequel le télomère comprend un télomère tête-à-tête.

6. Procédé selon la revendication 5, dans lequel le télomère tête-à-tête a un rendement supérieur à 60 % en poids.

7. Procédé selon la revendication 1, ou selon l'une quelconque des revendications 2 à 4, dans lequel le précurseur de catalyseur actif est Pd(OAc)₂ ; le ligand est la triphénylphosphine ; et le solvant comprend le trifluoroéthanol, et dans lequel le télomère comprend un télomère tête-à-tête.

8. Procédé selon la revendication 7, dans lequel le télomère tête-à-tête a un rendement supérieur à 60 % en poids.

9. Procédé selon la revendication 1, ou selon l'une quelconque des revendications 2 à 4, dans lequel le précurseur de catalyseur actif est [Pd(C₃H₅)COD]BF₄ ; le ligand est le tris(2,4-di-tert-butylphényl)phosphite ; et le solvant comprend l'éther diéthylique, le tétrahydrofurane ou le toluène, et dans lequel le télomère comprend un télomère queue-à-queue.

10. Procédé selon la revendication 9, dans lequel le télomère queue-à-queue a un rendement supérieur à 60 % en poids.

11. Procédé selon la revendication 1, ou selon l'une quelconque des revendications précédentes, dans lequel le système catalytique actif contient en outre un nucléophile.

12. Procédé selon la revendication 11, dans lequel le nucléophile comprend une amine.
